# EUROPEAN PATENT APPLICATION

(11) **EP 1 535 589 A1**
(43) Date of publication of application: **01.06.2005**
(21) Application number: 03794060.8
(22) Date of filing: 04.07.2003
(51) Int. Cl.: A61F 5/44, A61F 13/15, A61F 13/47

(54) **ABSORPTIVE PRODUCT**

(30) Priority: 29.08.2002 JP 2002251466
(71) Applicant: Pigeon Corporation, Tokyo 101-0043 (JP)
(72) Inventor: TSUTSUI, Katsushi, Chiyoda-ku, Tokyo 101-0043 (JP)
(74) Representative: Kremer, Simon Mark
(86) International application number: PCT/JP2003/008540
(87) International publication number: WO 2004/021937

(57) **Abstract**

An absorptive product can form and hold a stereoscopic shape which is properly formed to be snugly fitted on a complicated three-dimensional shape of a user's body to prevent leaking of an evacuated body fluid or the like to the outside of the body.

The absorptive product includes a back sheet which has a shape elongated in one direction and prevents the permeation of liquid, a liquid permeable surface material (28) which is arranged on a surface side which is brought into contact with a body, and an absorbent (23), (33) which is arranged between the back sheet and the surface material and absorbs and holds the liquid which permeates the surface material. The absorptive product further includes a resilient body (45) which is fixed at least to the absorbent side in a center region in the lateral direction T of the product and imparts a contracting force with respect to the arrangement direction, and slits (46), (46) which are formed in the absorbent in the vicinity of a region on which the contracting operation of the resilient body acts.

## Description

### Technical Field

The present invention relates to an absorptive product which is applicable to, for example, a disposable diaper, a sanitary napkin, an incontinence pad for absorbing and holding a body fluid such as urine and a manufacturing method thereof.

### Background Art

Conventionally, an absorptive product such as an incontinence pad, a sanitary napkin or the like is generally a pad body which is formed in a rectangular shape or an oblong shape elongated in one direction. By mounting the absorptive product at a position corresponding to a crotch part of an underwear in a state that the lengthwise direction of the absorptive product is adjusted in the fore-and-aft direction, the absorptive product can receive excrement such as urine, feces, menstrual blood which a user evacuates.

In such an absorptive product, a liquid-impermeable sheet material is applied to a portion which forms an outer surface when the user wears the absorptive product, an absorbing material for absorbing liquid such as urine is arranged inside the liquid-impermeable sheet material, and a liquid-permeable sheet material which allows the liquid to pass therethrough is arranged on a portion which is arranged inside the absorbing material and is brought into contact with a skin of the user.

Due to such a constitution, the urine or the like which forms the excrement permeates the sheet material which allows the liquid to pass therethrough and is absorbed by the absorbing material. Further, it is possible to prevent the soaking of the urine or the like to the outside by the liquid-impermeable sheet material.

However, the conventional absorptive product such as incontinence pad cannot sufficiently follow or come into contact with a profile of a lower half of user's body which has a complicated unevenness and hence, a gap is formed between the absorptive product and the body of the user thus giving rise to a drawback that the excrement leaks from this gap or the like.

Particularly, the incontinence pad or the like which is popularly used adopts the so-called crotch gather structure which raises both side portions or the like. However, only the use of this crotch gather structure is insufficient. Rather, it is ideal to provide the structure which allows a liquid absorbing portion of an absorptive product to be properly fitted on a part of a user corresponding to the change of posture such as a state in which the user is standing or a state in which the user is sleeping.

However, although various proposals have been made with respect to the absorptive product having such a stereoscopic shape (i.e. three dimensional conformation), when the absorptive product is folded or packaged before use, the stereoscopic shape is collapsed or as the user keeps wearing the absorptive product, the absorptive product cannot maintain the sufficient stereoscopic shape. Accordingly, there has been a demand for an absorptive product which can form and maintain the ideal stereoscopic shape.

Accordingly, it is an object of the present invention to provide an absorptive product such as an incontinence pad, a sanitary napkin or the like which can form and hold a proper stereoscopic shape so as to be properly fitted on a complicated three-dimensional shape of the body of a user for preventing leaking of an evacuated body fluid or the like to the outside.

### Disclosure of the Invention

The above-mentioned object can be achieved, according to the first aspect of the invention, by an absorptive product which includes a back sheet which has a shape elongated in one direction and prevents the permeation of liquid, a liquid permeable surface material which is arranged on a surface side which is brought into contact with a body, and an absorbent which is arranged between the back sheet and the surface material and absorbs and holds the liquid which permeates the surface material, wherein the absorptive product includes a resilient body which is fixed at least to the absorbent side in a center region in the lateral direction of the product and imparts a contracting force with respect to the arrangement direction, and slits which are formed in the absorbent in the vicinity of a region on which the contracting operation of the resilient body acts.

According to the first aspect of the invention, the absorptive product of the present invention has the basic shape which is elongated in one direction by stacking and fixing a plurality of sheet-like materials. The back sheet prevents the permeation of the liquid. The surface material is the liquid-permeable material which is arranged on the side which is brought into contact with the body. The absorbent is arranged between the back sheet and the surface material and absorbs and holds the liquid which permeates the surface material.

Further, according to the first aspect of the invention, the stereoscopic shape which conforms to the body of the user is formed and held using the resilient body. That is, the resilient body is, in the stacked structure which constitutes the product, particularly fixed to the absorbent side to impart the contracting force mainly to the absorbent to form the stereoscopic shape. In this case, the absorbent has a relatively large thickness, is hardly deformed and, further, is liable to easily receive the influence attributed to the absorption of the body fluid. By forming the slits in the absorbent, the contracting force of the resilient body acts as a deformation force to deform the absorbent such that the slit portions of the absorbent are bent and hence, the stereoscopic shape can be effectively formed and the formed shape can be maintained. Particularly, by arranging the resilient body at the center region in the lateral direction of the product, it is possible to provide the shape which conforms to a complicated shape of human body in the vicinity of a groin of the user. Accordingly, even when the body of the user is moved, the absorptive product can be deformed following the movement of the body and hence, leaking of liquid or the like can be surely prevented.

The second aspect of the invention is an embodiment of the first aspect of the invention, characterized in that the resilient body is arranged such that the resilient body imparts the contracting force to the absorbent mainly along the longitudinal direction of the product, and the slits are respectively arranged at both sides with respect to the resilient body in the lateral direction of the product.

According to the second aspect of the invention, the resilient body is arranged along the longitudinal direction of the product. Further, bent portions are formed on the material which constitutes the product at the slit forming portions which are arranged on both sides of the resilient body, and a portion to which the resilient body is fixed is effectively formed into a projecting shape due to the contracting force of the resilient body. Particularly, by forming the slits on both sides of the resilient body which are arranged outside the resilient body, the slits do not receive the influence of an adhesive agent such as a hot-melt which is attached to the resilient body and hence, the deformation is surely enhanced.

The third aspect of the invention is an embodiment of the second aspect of the invention, characterized in that the slits have at least one longitudinal end sides thereof parted away from the resilient body.

According to the third aspect of the invention, the slits are not formed in parallel to each other in the longitudinal direction of the product and at least one longitudinal end sides thereof are parted away from the resilient body and hence, it is possible to constitute the shape which conforms to the complicated shape of the human body. Further, at the time of forming the slits using a roller which forms a cutting blade on an outer periphery thereof in manufacturing steps, it is possible to obviate a case that a force is imparted to a limited portion of the roller and hence, it is possible to effectively prevent damage on the roller for forming the slits.

The fourth aspect of the invention is an embodiment of the second aspect of the invention or the third aspect of the invention, characterized in that the slits are formed on both sides of the resilient body such that one slit is formed on each side in symmetry and both slits have center portions thereof in the longitudinal direction thereof arranged close to each other and other portions thereof gradually parted away corresponding to the distance from the center portions.

According to the fourth aspect of the invention, when the slit portions which receive the contracting force of the resilient body are bent, the deformed region is configured such that a gentle curved surface where the portion to which the resilient body is fixed projects is formed and the curved surface is gradually flared downwardly whereby it is possible to easily form the shape which projects the center portion of the resilient body and to form the proper stereoscopic shape.

The fifth aspect of the invention is an embodiment of any one of the first aspect of the invention to the fourth aspect of the invention, characterized in that the resilient body is formed of a film-like resilient body having a given width which imparts a contracting force mainly in the longitudinal direction.

According to fifth aspect of the invention, compared to a case in which a linear rubber or the like is used as the resilient body, since the resilient body has the given width, the resilient body is brought into contact with a skin of the user who wears the absorptive product in a planar state whereby no undesired stimulus is given to the user thus reducing the discomfort which the user feels at the time of wearing the absorptive product. Further, the resilient body can exhibit the strong contracting force compared to the case in which the linear rubber or the like is used as the resilient body and hence, it is possible to impart the force sufficient to deform the absorbent which constitutes the fixed side.

The sixth aspect of the invention is an embodiment of any one of the first aspect of the invention to the fifth aspect of the invention, characterized in that the absorbent is formed by stacking a first absorbent layer having high liquid diffusivity of the absorbed liquid and a second absorbent layer having high liquid holding property of the absorbed liquid, the resilient body is fixed to the second absorbent layer, and the above-mentioned slits are formed in the second absorbent layer.

According to the sixth aspect of the invention, with the provision of at least two layers constituted of the first absorbent layer having high liquid diffusivity of the liquid and the second absorbent layer having high liquid holding property of the liquid, the absorptive product exhibits the excellent function that the absorptive product can speedily absorb, diffuse and hold the evacuated liquid component. Here, when the second absorbent layer is formed of a polymer sheet or the like, it is difficult to deform the second absorbent layer because it is relatively hard. However, by forming the slits in the second absorbent layer, the second absorbent layer becomes easily deformable and hence, the stereoscopic shape can be surely formed.

The seventh aspect of the invention is an embodiment of the sixth aspect of the invention, characterized in that a notched portion is formed in the first absorbent layer corresponding to a position where the resilient body is formed.

According to the seventh aspect of the invention, with respect to the region which is deformed by imparting of the contracting force of the resilient body, by removing a portion of the first absorbent layer having a relatively large thickness with the formation of the notched portion, it is possible to surely perform the deformation of the resilient body for forming the stereoscopic shape.

### Brief Description of the Drawings

Fig. 1 is a schematic perspective view of an incontinence pad according to the first embodiment of an absorptive product of the present invention.
Fig. 2 is a schematic plan view of the incontinence pad shown in Fig. 1.
Fig. 3 is a schematic cross-sectional view taken along a line A-A in Fig. 2.
Fig. 4 is a schematic cross-sectional view taken along a line B-B in Fig. 2.
Fig. 5 is an explanatory view showing a method for fixing a resilient body of the incontinence pad shown in Fig. 1.
Fig. 6 is an explanatory view showing a method for forming slits in the incontinence pad shown in Fig. 1.
Fig. 7 is an explanatory view showing a method for using the incontinence pad shown in Fig. 1.
Fig. 8 is an enlarged view of an essential part showing a modification 1 of the incontinence pad shown in Fig. 1.
Fig. 9 is an enlarged view of an essential part showing a modification 2 of the incontinence pad shown in Fig. 1.
Fig. 10 is an enlarged view of an essential part showing a modification 3 of the incontinence pad shown in Fig. 1.
Fig. 11 is an enlarged view of an essential part showing a modification 4 of the incontinence pad shown in Fig. 1.
Fig. 12 is a schematic plan view of an incontinence pad according to the second embodiment of an absorptive product of the present invention.
Fig. 13 is an explanatory view showing a method for fixing a resilient body of the incontinence pad shown in Fig. 12.

### Best Mode for carrying out the Invention

Preferred embodiments of the present invention are explained in detail hereinafter in conjunction with attached drawings.

Here, since the embodiments described hereinafter are preferred specific examples of the present invention, various limitations which are technically desirable are provided. However, the scope of the present invention is not limited to these embodiments unless there exist descriptions which particularly limit the present invention in the explanation made hereinafter.

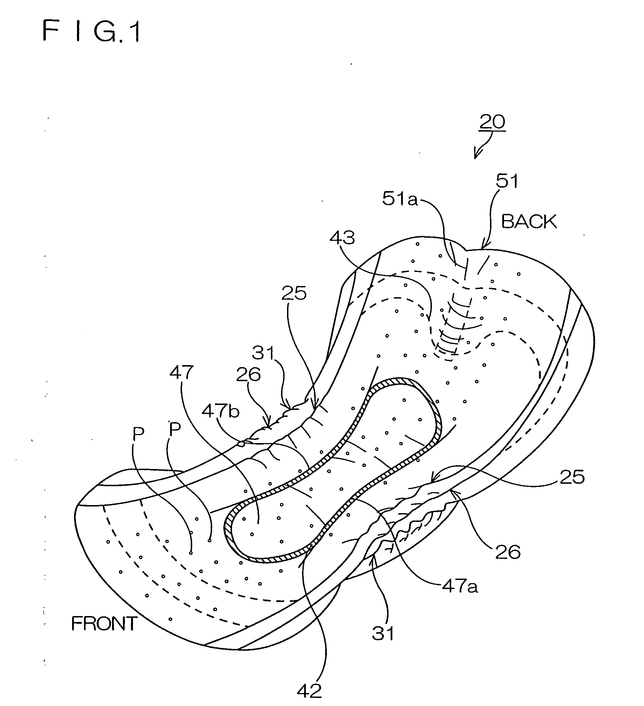

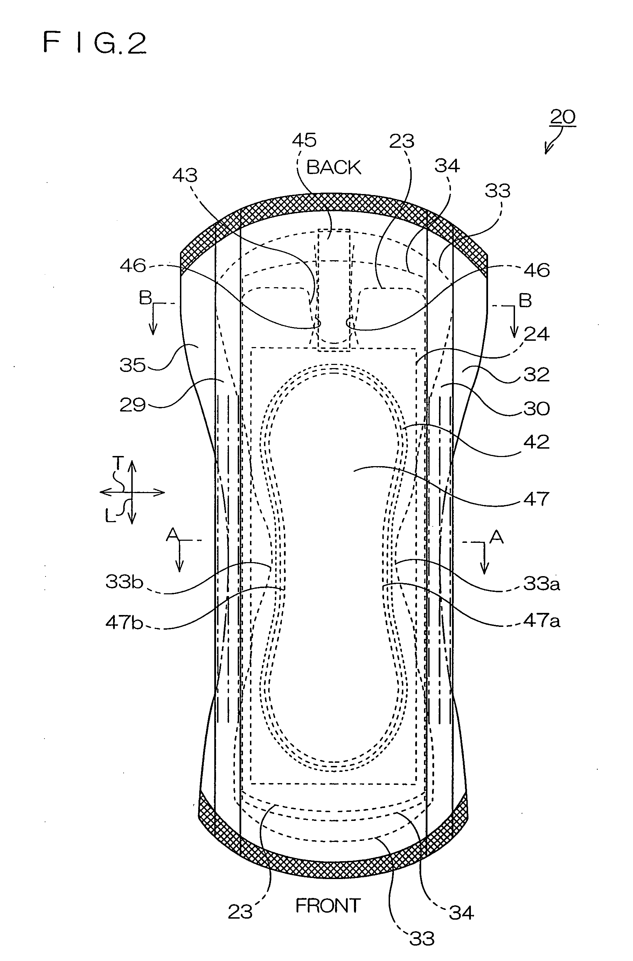

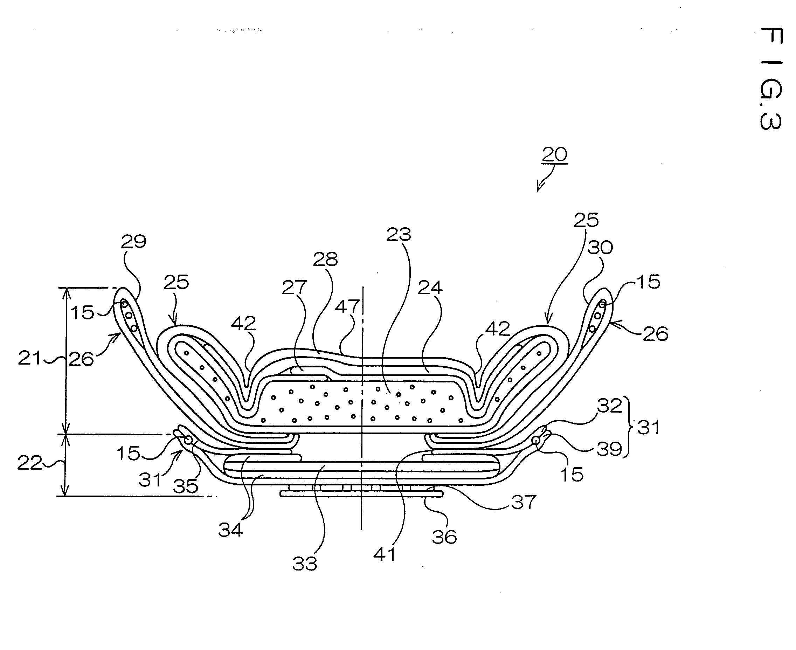

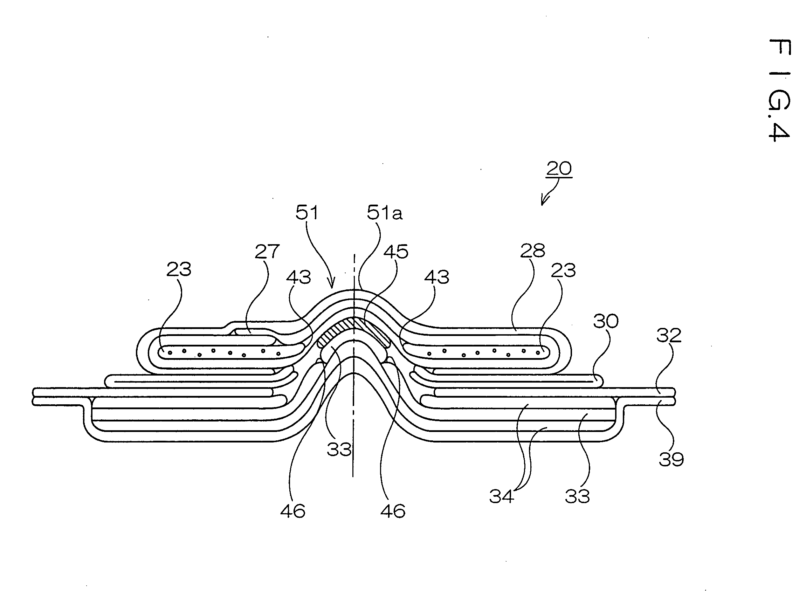

Fig. 1 to Fig. 4 show an absorptive product according to the first embodiment of the present invention and show the constitution of an incontinence pad as one example of the absorptive product. In this embodiment, although the explanation is made in view of an example in which the present invention is applied to the incontinence pad which represents the absorptive product, the present invention is applicable to a sanitary napkin or a diaper using the same structure by merely slightly changing the size of the whole structure. Here, Fig. 1 is a schematic perspective view of the incontinence pad 20 as viewed from a front side. In the drawing, a side which is indicated by RONT constitutes a front side of a body when a user wears the incontinence pad 20 (the same interpretation being applicable to other drawings). Fig. 2 is a schematic plan view of the incontinence pad 20 shown in Fig. 1. Fig. 3 is a schematic cross-sectional view taken along a line A-A in Fig. 2 and Fig. 4 is a schematic cross-sectional view taken along a line B-B in Fig. 2.

As shown in Fig. 1, the incontinence pad 20 has a shape which is elongated in the fore-and-aft direction. The incontinence pad 20 has, as shown in the drawing, a deformed portion 51 in the vicinity of a rear end portion. The deformed portion 51 is formed of the structure described later and is provided with a projecting portion 51a which extends in the longitudinal direction in the vicinity of the center thereof. Due to such a constitution, in a state that the user wears the incontinence pad 20 as described later, the deformed portion 51 enters a crevice of a hip of the user to fill a portion which constitutes a valley thus performing a function of eliminating a gap.

In Fig. 1, in the vicinity of the center of the inside of the incontinence pad 20, a region which is more or less elongated in the longitudinal direction is formed and this region constitutes a projecting portion 47, wherein the projecting portion 47 is properly brought into contact with a part of the user. The periphery of the projecting portion 47 is defined by a channel emboss portion 42 which is formed of a groove-like emboss. On both sides of the channel emboss portion 42, stereoscopic gather portions 26, 26 are raised to form barriers to prevent leaking of an evacuated liquid or the like sideward. Further, on both sides of the stereoscopic gather portions 26, 26, crotch gather portions 31 are formed and are brought into close contact with a crotch part of the user. Accordingly, even when a small amount of the evacuated liquid or the like leaks from the stereoscopic gather portions 26, 26, it is possible to prevent the evacuated liquid or the like from leaking to the outside.

Fig. 2 to Fig. 4 show the detailed structure which constitutes the above-mentioned respective portions. The structure of the incontinence pad 20 is explained in further detail in conjunction with these drawings. Here, the cross sections shown in Fig. 3 and Fig. 4 are depicted by magnifying a thickness of the incontinence pad 20 compared to an actual thickness of the incontinence pad 20 to facilitate the understanding of the structure.

As shown in Fig. 3, the incontinence pad 20 has the structure which is formed by overlapping and fixing a plurality of stacked bodies constituted of a first stacked body 21 and a second stacked body 22 from a side of the incontinence pad 20 which is brought into contact with the user's body.

The first stacked body 21 has the structure in which a first absorbent layer 23 which is an absorbent wrapped with a tissue 27 is covered with a center surface material 28 which is brought into contact with the user's body.

The tissue 27 and the first absorbent layer 23 are portions which are served for absorbing and holding the liquid or the like evacuated from the user.

As the tissue 27, it is possible to use a tissue which is obtained by forming a soft material which can absorb the liquid or the like such as, for example, pulp (virgin pulp, waste paper-reclaimed pulp), rayon, cotton, kenaf, bagasse, silk, hydrophilization-processed fibers (polyolefin-based, polyester, acrylic) into a sheet in a single form or in a composite form.

The tissue 27 covers the first absorbent layer 23 and prevents a portion of content from leaking or falling. As shown in Fig. 3 and Fig. 4, the tissue 27 is bonded in an overlapped manner at a portion offset from the center in the lateral direction T. Due to such a constitution, it is possible to arrange the tissue 27 such that the tissue 27 does not interrupt a contracting force of a resilient body 45 described later.

The first absorbent layer 23 constitutes a portion of the absorbent of this embodiment and mainly possesses the property to rapidly diffuse the absorbed liquid and, thereafter, to hold the absorbed liquid. The first absorbent layer 23 is referred to as a so-called "mat" material and is preferably formed of, for example, a pulp absorbent which is formed by mixing polymer which constitutes an absorbing material into pulp, a pulp absorbent which is formed by scattering the polymer into pulp or polymer or the like. Here, the pulp is an aggregate of cellulose fibers which are obtained by extracting after mechanically or chemically processing timbers, for example. In a state that a relatively large number of pores are present among fibers, the pulp promotes the diffusion of the absorbed liquid. Further, the pulp holds a large amount of polymer and hence, the volume in the thickness direction is increased whereby the absorbing capacity of the liquid can be increased.

Since the center surface material 28 is directly brought into contact with the skin of the user, a material suitable for the center surface material 28 is selected by considering that the material feels soft without excessively damaging the skin. The center surface material 28 is also referred to as an inner sheet, a top sheet or the like. As fibers of the material which are suitable for the permeation of liquid, particularly for the rapid permeation of the liquid component, for example, various natural fibers, synthetic fibers or the combination of these fibers can be selected. For example, as the synthetic fibers, fibers which use polyester or polypropylene fibers as a base material and are processed to increase the liquid permeability are preferable. For example, when the center surface material 28 is formed of nonwoven fabric, for example, it is possible to adopt wet nonwoven fabric (paper, tissue, hydro-span (product of Dexter Ltd.), chemical-bond (or resin-bond), thermal-bond (emboss, air-through), air-laid, span-lace, span-bond, melt-blown, needle punching, stitch-bond, SM nonwoven fabric (stacked body of span-bond and melt-blown) which is formed by combining span-bond and melt-blown nonwoven fabrics, SMS nonwoven fabric (stacked body of span-bond, melt-blown and span-bond) and the like. Among nonwoven fabrics, as the material of the center surface material 28, it is particularly preferable to use the thermal bond, the span-bond, and the span lacing.

Further, in this embodiment, on the side which is brought into contact with the user's body, a sub layer 24 is preferably formed between the center surface material 28 and the tissue 27. The sub layer 24 is formed of bulky nonwoven fabric (having a large thickness and high porosity) which is integrally formed using chemical fibers such as polypropylene, polyethylene, polyester or the like, for example. Further, in a state that the center surface material 28 is overlapped to the sub layer 24, by applying minute emboss forming (pin emboss) to both nonwoven fabrics constituted of the center surface material 28 and the sub layer 24 as indicated by symbol P in a scattered manner in Fig. 1, a diffusion sheet is formed. By providing the diffusion sheet, the incontinence pad 20 can perform the function of rapidly diffusing the liquid which permeates the center surface material 28 to the first absorbent layer 23 side and, further, the function of preventing the liquid or the like which is absorbed by the first absorbent layer 23 from moving toward the body side.

Between the diffusion sheet and the lowermost layer of the first stacked body 21, that is, the second stacked body 22, two side surface materials 29, 30 are arranged along both side peripheries thus forming stereoscopic gather portions 26.

That is, the side surface materials 29, 30 are arranged on both sides of the center surface material 28 along the longitudinal direction L of the center surface material 28 (the direction L being indicted in Fig. 2). While the center surface material 28 allows the liquid to permeate therethrough, the side surface materials 29, 30 are made of a material which prevents the permeation of the liquid.

As the material of the side surface materials 29, 30, for example, the combination of polyolefin-based nonwoven fabric or the polyester-based nonwoven fabric and a thin plastic film or the combination of the polyester-based nonwoven fabric and a polyethylene film or the like, the SM nonwoven fabric, the SMS nonwoven fabric and the like can be used.

The left and right stereoscopic gather portions 26, 26 are formed by arranging resilient bodies 15 respectively on at least the center portions in the longitudinal direction of the side surface materials 29, 30. Due to the resilient contracting ability of the resilient bodies 15, as shown in Fig. 2, the stereoscopic gather portions 26 are formed on at least the center portions (folded-back portions in a pouch shape) of the folded respective side surface materials 29, 30. By forming the stereoscopic gather portions 26, the stereoscopic gather portions 26 are brought into close contact with the user's body to prevent the excrement or the liquid component of the user from leaking to the outside along the widthwise direction (direction orthogonal to the longitudinal direction L in Fig. 2) T of the incontinence pad 20.

The constitution of the second stacked body 22 shown in Fig. 3 is explained.

At the center of the second stacked body 22, a second absorbent layer 33 is arranged. The second absorbent layer 33 is arranged outside the first stacked body 21 which is provided with the first absorbent layer 23 and plays a role of absorbing and holding the liquid or the like which the first absorbent layer 23 cannot absorb and hold. Accordingly, the second absorbent layer 33 has the high absorbed-liquid holding property and also has the relatively thin non-bulky configuration. As this second absorbent layer 33, for example, pulp in a single form or, if necessary, a pulp absorbent which is formed by mixing polymer which constitutes an absorbing material in pulp or by scattering the polymer in the pulp can be used. However, it is particularly preferable to use a polymer sheet which is formed in a sheet form by arranging polymer with high density between sheets made of pulp or the like. Here, the pulp is a material having a small thickness which is obtained by densely bonding an aggregate of cellulose fibers which are obtained by extracting after mechanically or chemically processing timbers using an adhesive agent or the like, for example. Accordingly, the second absorbent layer 33 has the high hardness, the high rigidity and the high shape holding property compared to the first absorbent layer 23.

The second absorbent layer 33 is covered with a tissue 34 for preventing the dissipation of the polymer or the like. Further, a back sheet 39 is arranged on the outside of the tissue 34. The back sheet 39 constitutes an outer sheet material and is made of a material which can prevent the permeation of the liquid. As the material of the back sheet 39, for example, a thin plastic film, to be more specific, a polyethylene film, a polyethylene laminate paper or the like can be used. As the material of the back sheet 39, it may be possible to select a material which prevents the permeation of liquid, allows the permeation of vapor to prevent the stuffy condition, and has flexibility to some extent. Due to such a selection, it is possible to prevent the content inside the incontinence pad 20 (excrement such as feces or urine, blood or the like) from leaking to the outside.

Further, in Fig. 3, between the tissue 34 and the respective side surface materials 29, 30 of the first stacked body 21, crotch gather nonwoven fabrics 32, 35 are fixed. That is, the crotch gather nonwoven fabrics 32, 35 respectively have the strip-like configuration and are arranged and fixed to both sides of the product along the longitudinal direction L. By forming openings 41 between the crotch gather nonwoven fabrics 32, 35, the liquid or the like from the first stacked body 21 is allowed to pass therethrough toward the second stacked body 22 side. Here, a thickness of the opening 41 shown in Fig. 3 is expressed to facilitate the understanding and, in an actual constitution, the tissue 27 and the second absorbent layer 33 are bonded to each other.

By adhering respective outer side peripheral portions of these crotch gather nonwoven fabrics 32, 35 to respective side peripheral portions of the back sheet 39, crotch gather portions 31, 31 are formed. That is, by inserting the string-like resilient bodies 15, 15 into the adhering portions between the respective outer side peripheral portions of these crotch gather nonwoven fabrics 32, 35 to respective side peripheral portions of the back sheet 39, the gather structure similar to the stereoscopic structure is formed.

The crotch gather portions 31 pull both end portions in the lateral direction which correspond to the crotch of the incontinence pad 20 in a cooperative manner with the stereoscopic gather portions 26 due to the resilient force and hence, the fit feeling of the incontinence pad 20 on the crotch of the user is enhanced, the incontinence pad 20 can exhibit the function of preventing the leaking of the excrement or the liquid component of the user from the crotch part.

Further, an adhesive portion 37 for temporary fixing is formed on an outer surface of the back sheet 39 and a peeling sheet 36 is laminated to the adhesive portion 37.

Due to such a constitution, when the incontinence pad 20 is used, the peeling sheet 36 is peeled off to expose the displacement preventing adhesive portion 37. Since the displacement preventing adhesive portion 37 is replaceably adhered to and laminated to an inner surface of an underwear or the like, for example, it is possible to fix the incontinence pad 20 to the underwear such as underpants without displacement. Here, both end portions at the center region of the longitudinal direction L of the back sheet 39 may be further extended, adhesive portions may be formed on lower surfaces of both end portions, and the extended portions may be folded back and fixed to outer surface of the underwear thus forming so-called wing portions.

Further, in the incontinence pad 20 of this embodiment, as shown in Fig. 1 and Fig. 3, the first absorbent layer 23 has high fluid absorbing and diffusing ability and also has the large thickness and hence, the inner region defined by the channel emboss portion 42 which constitutes the projecting portion 47 is brought into close contact with the part of the user's body. Accordingly, the gap is not formed with respect to the part of the body and hence, the leaking of the evacuated liquid or the like sideward and the like can be prevented and the evacuated liquid or the like can be speedily absorbed. Particularly, with respect to the projecting portion 47, as shown in Fig. 1 and Fig. 2, in the vicinity of the center in the longitudinal direction of the incontinence pad 20, narrowed-width recessed portions 47a, 47b are formed and the channel emboss portions 42 also have the similar shape. Further, as shown in Fig. 2, corresponding to such a structure, also with respect to the second absorbent layer 33, narrowed-width recessed portions 33a, 33b are formed in the vicinity of the center in the longitudinal direction of the incontinence pad 20. In this manner, the projecting portion 47 is configured to conform to the shape of the crotch part of the user's body and hence, the incontinence pad 20 fits on the user's body more properly thus preventing the leaking of evacuated liquid sideward or the like.

Further, corresponding to this structure, as shown in Fig. 1 and Fig. 3, both side portions of the region which is provided with the first absorbent layer 23 having the large thickness are arranged inside the stereoscopic gather portions 26, 26 and hence, both side portions of the region constitute side barrier portions 25, 25 which are raised due to the erecting action of the stereoscopic gather portions 26, 26. Accordingly, in addition to the above-mentioned action of the stereoscopic gather portions 26, 26, the liquid or the like which is considered to flow sideward (the direction T in Fig. 2) can be effectively absorbed inside the stereoscopic gather portions 26, 26 and hence, it is possible to prevent the leaking of the evacuated liquid or the like sideward.

Further, the incontinence pad 20 of this embodiment includes the above-mentioned deformed portion in the region at one end of the longitudinal direction L of the product shown in Fig. 2, for example, in the vicinity of a rear end portion of the product in the case shown in the drawing. The deformed portion 51 has, as shown in Fig. 1 and Fig. 2, the structure which includes slits 46, 46 formed in the second absorbent layer 33 with a given width therebetween from the vicinity of the end portion of the second absorbent layer 33 arranged at the rear end of the incontinence pad 20 and the vicinity of the channel emboss portion 42.

Although the number of the slits 46, 46 formed in the second absorbent layer 33 may be one, it is preferable to form two slits 46, 46 as shown in the drawing.

Due to such a constitution, when the slit portions 46, 46 which receive the contracting force of the resilient body 45 are bent as shown in Fig. 1, the deformed portion 51 is configured , as shown in Fig. 4, such that a gentle curved surface 51a where the portion to which the resilient body 45 is fixed projects upwardly is formed and the curved surface is gradually flared downwardly from the gentle curved surface 51a whereby it is possible to easily form the stereoscopic shape which is hardly collapsed.

Here, the slits 46, 46 are not always necessary to be formed of an interstice which completely cuts the second absorbent layer 33 and may be formed of a groove-like slit having a narrow width, a sawing-stitch-like slit, a slit which does not completely cut the second absorbent layer 33 or the like.

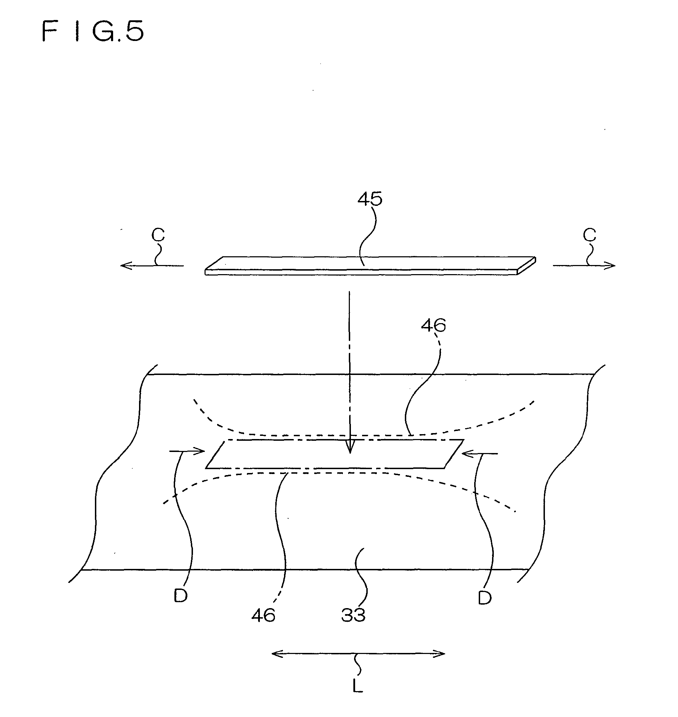

Fig. 5 shows the manner of fixing the resilient body 45.

Here, as the resilient body 45, as shown in the drawing, for example, a film-like resilient body having a given width which imparts a contracting force mainly in the longitudinal direction is suitably used.

In a state that a tension is applied to the film-like resilient body 45 in arrow directions C, C as shown in the drawing, the resilient body 45 is fixed by lamination to the second absorbent layer 33 using an adhesive agent such as a hot-melt. Here, Fig. 5 is an explanatory view and hence, the shape of the second absorbent layer 33 is not equal to an actual shape.

Due to such a constitution, with respect to the second absorbent layer 33, to a portion thereof to which the resilient body 45 is fixed, the resilient body 45 imparts the contracting force indicted by arrows D, D with respect to the longitudinal direction L of the incontinence pad 20 and this force acts as a deformation force which bends the slits 46, 46 upwardly.

Here, the film-like resilient body made of urethane, polyethylene, natural rubber or the like is used as the resilient body 45. Accordingly, by adopting such a structure, compared to a case in which a linear rubber or the like is used, it is possible to bring a portion of the second absorbent layer 33 where the resilient body 45 is arranged into direct contact with the skin of the user who wears the incontinence pad 20 in a planar state, no undesired stimulus is given to the user and a discomfort that the user feels when the user wears the incontinence pad 20 can be reduced. Further, since the film-like resilient body 45 exhibits the strong contracting force compared to a case in which a linear rubber or the like is used as the resilient body, it is possible to impart the force sufficient to deform the second absorbent layer 33 which constitutes the fixed side.

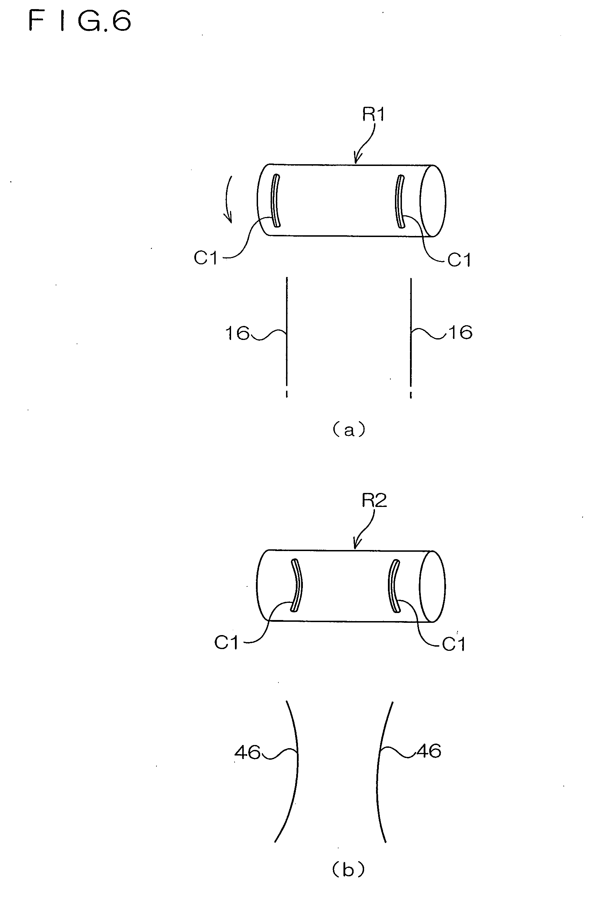

Further, although the slits 46, 46 may be formed in parallel to each other, in forming the parallel slits, there arises a drawback shown in Fig. 6(a) in manufacturing steps. A roller cutter R1 shown in Fig. 6(a) is provided with blades C1, C1 which are arranged in parallel to each other on an outer periphery of the roller. To form the slits by cutting the second absorbent layer 33 using such a roller cutter R1, parallel slits 16, 16 shown in a lower drawing are formed.

However, when such forming is continued, a forming load is applied to some specific portions of the roller cutter R1 where the blades C1, C1 are provided and hence, the roller cutter R1 is liable to be easily damaged whereby not only it pushes up a cost for exchange, but also a manufacturing line has to be stopped often thus lowering the productivity.

To the contrary, with respect to a roller cutter R2 shown in Fig. 6(b), blades C1, C1 are formed on an outer periphery of the roller in a non-parallel state such that the blades C1, C1 are arranged close to and away from each other. When the slits are formed by cutting the second absorbent layer 33 using such a roller cutter R2, the slits 46, 46 shown in a lower drawing are formed. That is, the slits 46, 46 are not arranged parallel to the longitudinal direction L of the incontinence pad 20 and are formed such that at least one-end sides thereof are parted away from the resilient body 45 arranged between the slits 46, 46. Particularly, in this embodiment, the slits 46, 46 are formed on both sides of the resilient body 45 such that one slit is formed on each side in symmetry and both slits 46, 46 have center portions thereof in the longitudinal direction thereof arranged close to each other and other portions thereof gradually parted away corresponding to the distance from the center portions. That is, for example, the slits 46, 46 are formed in an arcuate shape respectively.

Due to such a constitution, it is possible to prevent the imparting of the force on the limited portions and hence, the damage on the roller cutter R2 for forming the slits can be effectively prevented. Further, by forming these two slits in an arcuate shape, the second absorbent layer 33 can be easily deformed in a state that the center portion having the narrow width projects whereby the second absorbent layer 33 can easily conform to the shape of the body.

The slits 46, 46 formed in this manner are formed in the second absorbent layer 33. Since the second absorbent layer 33 contains a large amount of polymer, the second absorbent layer 33 not only easily receives the influence of the liquid but also is relatively hard and hence is difficult to be deformed. Accordingly, by providing the slits 46, 46 to the second absorbent layer 33, the second absorbent layer 33 can be easily deformed and the stereoscopic shape can be surely formed.

Further, as shown in Fig. 2, corresponding to the position where the resilient body 45 is formed, notched portions 43 are formed in a rear end portion of the first absorbent layer 23. Accordingly, with respect to regions which are to be deformed by imparting the contracting force of the resilient body 45, by removing portions of the first absorbent layer 23 having a relatively large thickness by forming the notched portions 43, it is possible to enhance the deformation attributed to the resilient body 45 to be directed toward the body side and hence, it is possible to perform the deformation for forming the stereoscopic shape more reliably.

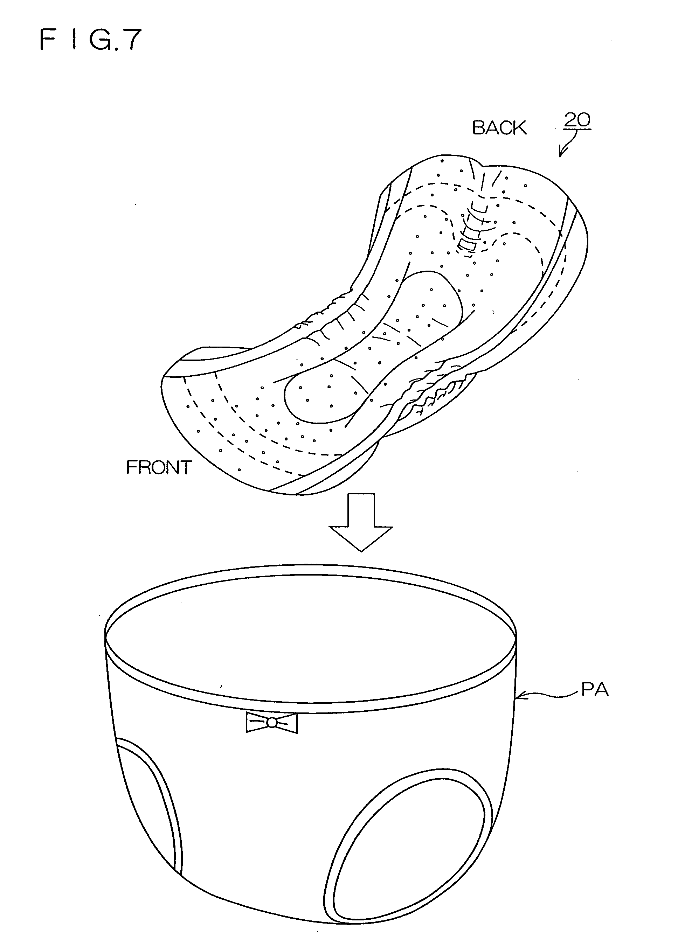

The incontinence pad 20 of this embodiment has the above-mentioned constitution and, as shown in Fig. 7, the incontinence pad 20 is temporarily fixed to a portion of the inside of an underwear PA corresponding to a crotch part by making use of the above-mentioned adhesive portion 37 for temporarily fixing (see Fig. 3) in a state that the deformed portion 51 is deformed. In Fig.7, for facilitating the understanding, compared to the size of the underwear PA formed of an underpant, a paper diaper or the like, the incontinence pad 20 is described larger than an actual size. Further, the incontinence pad 20 includes the above-mentioned deformed portion 51 in the vicinity of the rear end thereof. The deformed portion 51 enters the crevice of the user's hip and is filled in the portion which constitutes a valley whereby a gap can be eliminated.

Accordingly, even when the user works while standing in a state that the user wears the incontinence pad 20 or the user sleeps while wearing the incontinence pad 20, there is no gap between the portion of the crevice of the hip and the incontinence pad 20 and hence, there is no possibility that the urine or the like leaks therefrom. Further, since the deformed portion 51 enters the crevice of the hip, it is possible to prevent the occurrence of displacement and twisting when the user moves the body whereby the absorbing property can be surely exhibited and there is no fear that the clothing such as the underwear is smeared. Further, the above-mentioned stereoscopic shape can be properly formed and, at the same time, it is possible to hold the stereoscopic shape without damaging the stereoscopic shape even in a state that the user wears the incontinence pad 20 or in a state that the user carries the incontinence pad 20.

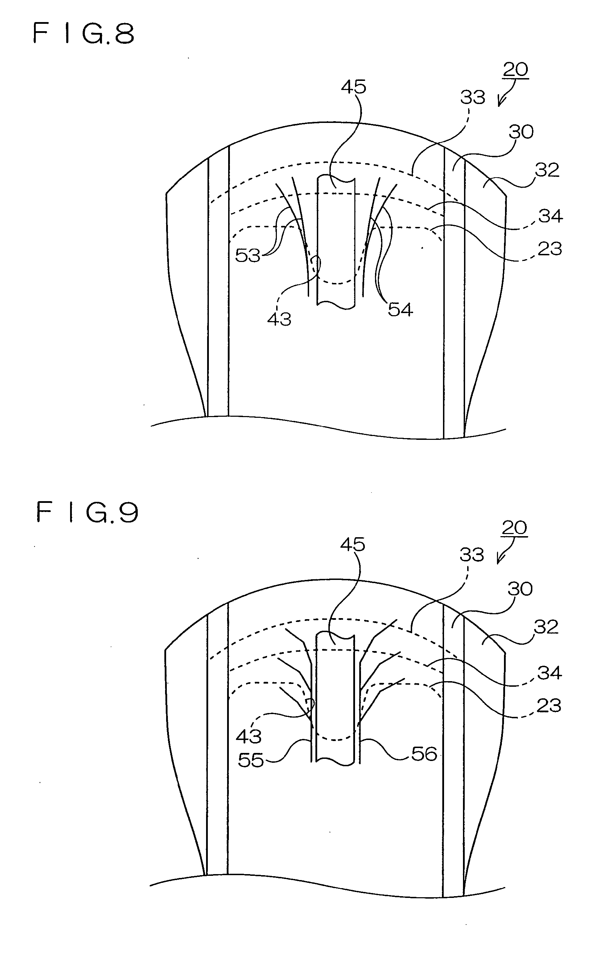

Fig. 8 to Fig. 11 show respective modifications of the first embodiment and these drawings show only the vicinity of the rear end portion of the incontinence pad 20. In these modifications, parts which are given symbols equal to the symbols used in the explanation of the above-mentioned first embodiment and parts omitted from the drawing are constitutions which are used in common in the explanation of the first embodiment and hence, the duplicate explanations are omitted and only points which make these modifications different from the first embodiment are explained.

The modifications 1, 2 shown in Fig. 8 and Fig. 9 are respectively examples of other slits which are formed on the second absorbent layer 33. Both slits are formed in a state that a lower portion thereof is narrowed and an upper portion is widened in the drawings.

That is, in the modification 1 shown in Fig. 8, with respect to respective slits 53, 54 formed on both sides of the resilient body 45 in a state that the slits 53, 54 sandwich the resilient body 45 therebetween, the lower portion is formed of a single slit and the upper portion is formed of two divided slits and these slits are expanded outwardly as the slit extends upwardly. The above-mentioned slits constitute portions which are bent and deformed by receiving a contracting force of the resilient body 45. In this case, corresponding to a fact that a profile of the hip of the user who wears the incontinence pad 20 has an approximately spherical stereoscopic shape from a lower portion to an upper portion, by forming two bending slit portions respectively, the deformed portion 51 can be deformed in a more stereoscopic configuration to conform to a curved surface of the hip.

In the modification 2 shown in Fig. 9, with respect to respective slits 55, 56 formed on both sides of the resilient body 45 in a state that the slits 55, 56 sandwich the resilient body 45 therebetween, the lower portion is formed of a single slit and the upper portion is formed of three divided slits. Also by forming three bending slit portions respectively, the deformed portion 51 can be deformed in a more stereoscopic configuration to conform to a curved surface of the hip. Further, since three slits are inclined such that proximal portions thereof are radially formed and the vicinities of the distal end portions thereof are inclined while extending in the oblique direction in parallel thus providing the configuration which also conforms to a curved surface of the body in the fore-and-aft direction.

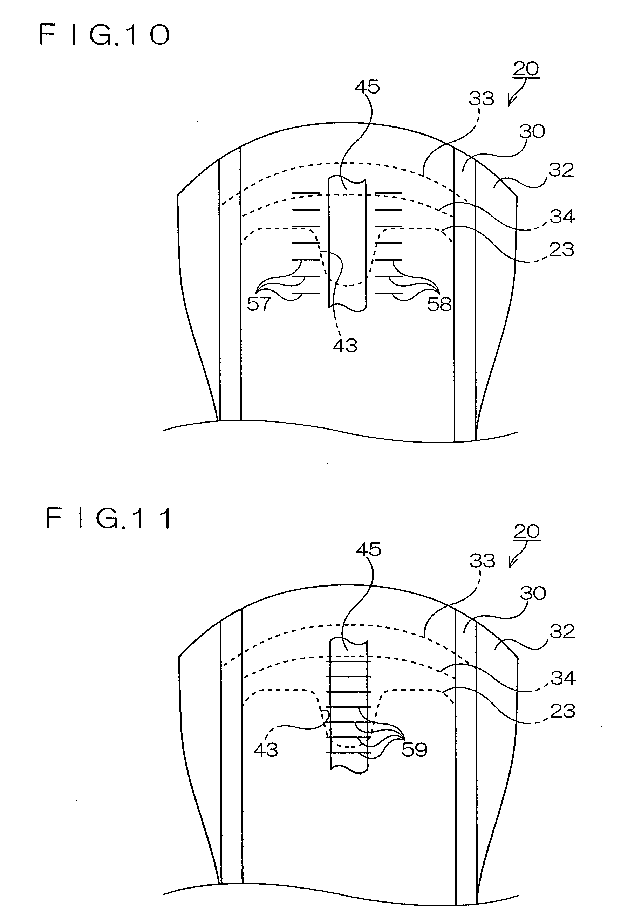

Both of the modification 3 and the modification 4 shown in Fig. 10 and Fig. 11 show examples in which slits are formed along the widthwise direction T which is orthogonal to the longitudinal direction L of the incontinence pad 20.

In the modification 3 shown in Fig. 10, with respect to respective slits 57, 58 formed on both sides of the resilient body 45 in a state that the slits 57, 58 sandwich the resilient body 45 therebetween, each slit 57 is constituted of a large number of short slits which are arranged along the horizontal direction in the drawing and parallel to the longitudinal direction of the incontinence pad 20.

Due to such a constitution, by bending the respective slits, the incontinence pad 20 is configured to particularly conform to a curved surface of a user's body in the fore-and-aft direction without obstructing the deformation attributed to the contraction of the resilient body 45.

In the modification 4 shown in Fig. 11, a large number of short slits which extend in the horizontal direction and are arranged parallel in the longitudinal direction of the incontinence pad 20 are formed on the resilient body 45 in an overlapped manner without being arranged on both sides of the resilient body 45 in a state that both end portions of each slit respectively project from the resilient body 45. The slits may be provided at a position where the slits are overlapped to the resilient body 45. In this case, it is also possible to obtain the substantially equal manner of operation and advantageous effects as those of the third embodiment.

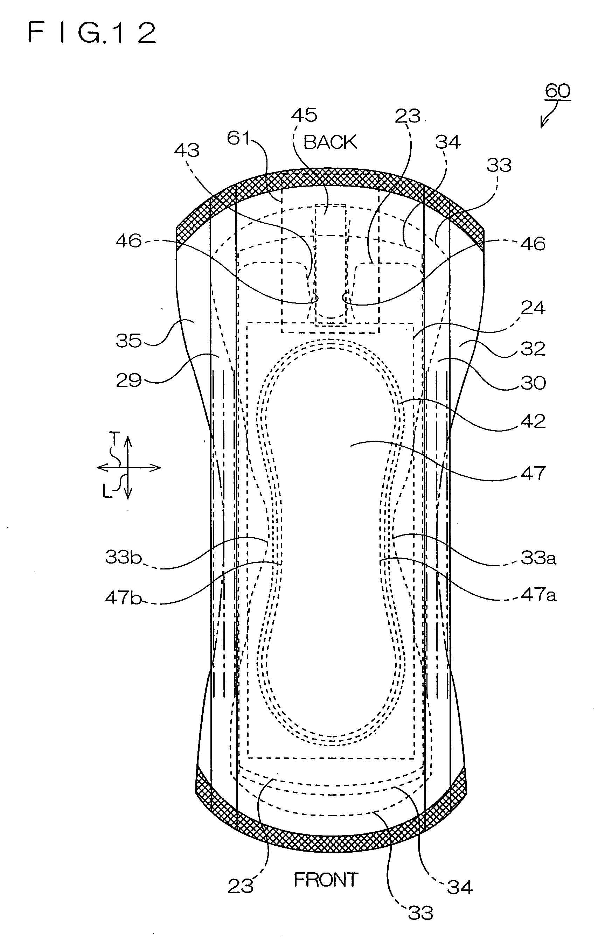

Fig. 12 shows an incontinence pad 60 of the second embodiment of the absorptive product of the present invention. Fig. 12 corresponds to Fig. 2 of the first embodiment. In the second embodiment, parts which are given symbols equal to the symbols used in the first embodiment have the common constitutions and also exhibit the same manner of operation and advantageous effects and hence, the duplicate explanation is omitted and only points which make this embodiment different from the first embodiment are explained.

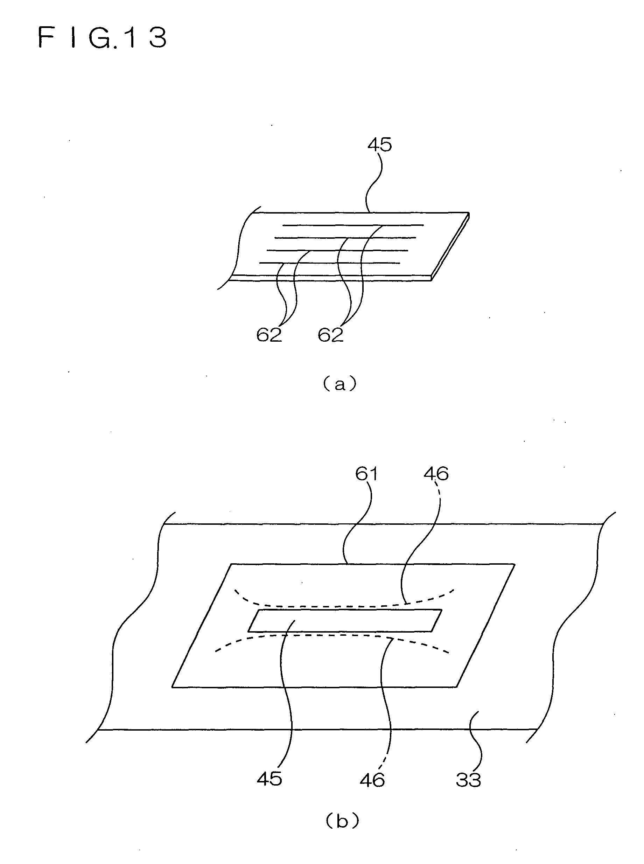

As shown in Fig. 13(a), in this embodiment, adhesive agents 62, 62 such as hot-melt or the like, for example, are applied to the resilient body 45 and, as shown in Fig. 13(b), a nonwoven fabric 61 is laminated to a surface of the resilient body 45 to which the adhesive agents are applied. The resilient body 45 is fixed to the second absorbent layer 33 side by laminating the nonwoven fabric 61 to the second absorbent layer 33.

The above-mentioned nonwoven fabric 61 is fixed to a portion indicated by symbol 61 in Fig. 12.

The reason that such a constitution is adopted is as follows. When the second absorbent layer 33 is directly laminated to the resilient body 45 as in the case of the first embodiment, depending on the contracting force of the resilient body 45 or depending on the structure of the second absorbent layer 33, an adhesive portion between the resilient body 45 and the second absorbent layer 33 peels off a surface material of the second absorbent layer 33 and, eventually, the resilient body 45 is peeled off from the second absorbent layer 33.

Accordingly, the resilient body 45 is laminated to the nonwoven fabric 61 whose rigidity is decreased to an extent that the nonwoven fabric 61 does not influence the contracting force of the resilient body 45 and, further, the nonwoven fabric 61 having a large area is laminated to the second absorbent layer 33 and hence, the contracting force of the resilient body 45 is focused on a small area of the second absorbent layer 33 whereby the peeling of the surface layer which is liable to be easily peeled can be effectively prevented. Here, slits equal to the slits formed in the second absorbent layer 33 may be also formed in the nonwoven fabric 61. Further, the resilient body 45 may be arranged such that a plurality of linear rubbers are formed in parallel or the nonwoven fabric 61 may be arranged such that the contracting force of the resilient body 45 acts in the lateral direction T different from the direction L. Other manner of operation and advantageous effects of the present invention are equal to those of the first embodiment.

Here, the present invention is not limited to the above-mentioned embodiments.

Although the incontinence pad is illustrated as the example of the absorptive product in the above-mentioned embodiments, the present invention is not limited to this incontinence pad and is applicable to absorptive products other than the incontinence pad, for example, various other products including menstrual articles, diapers and the like.

Further, although explanation has been made with respect to the example in which the absorbent has the two-layered structure in the above-mentioned embodiments, the absorbent may be constituted of a single layer or may be formed of a multi-layered absorbent having layers greater than two layers.

Still further, although the explanation has been made with respect to the case in which the deformed portion 51 is provided at the portion corresponding to the hip in the above-mentioned embodiments, the deformed portion 51 may be formed in the crotch part which is the center region along the longitudinal direction L.

The individual constitutions of the above-mentioned respective embodiments may be omitted if necessary and may be combined with other constitutions not explained in the specification.

### Industrial Applicability

As has been explained heretofore, the present invention is applicable to the disposable diaper, the sanitary napkin, the incontinence pad or the like, for example, and can be preferably used for the absorptive product for absorbing and holding the body fluid such as urine and the manufacturing method of the absorptive product.

## Claims

1. An absorptive product comprising:
a back sheet which has a shape elongated in one direction and prevents the permeation of liquid;
a liquid permeable surface material which is arranged on a surface side which is brought into contact with a body; and
an absorbent which is arranged between the back sheet and the surface material and absorbs and holds the liquid which permeates the surface material,
wherein the absorptive product includes
a resilient body which is fixed at least to the absorbent side in a center region in the lateral direction of the product and imparts a contracting force with respect to the arrangement direction, and
slits which are formed in the absorbent in the vicinity of a region on which the contracting operation of the resilient body acts.

2. An absorptive product according to claim 1, wherein the resilient body is arranged such that the resilient body imparts the contracting force to the absorbent mainly along the longitudinal direction of the product, and the slits are respectively arranged at both sides with respect to the resilient body in the lateral direction of the product.

3. An absorptive product according to claim 2, wherein the slits have at least one longitudinal end sides thereof parted away from the resilient body.

4. An absorptive product according to claim 2 or 3, wherein the slits are formed on both sides of the resilient body such that one slit is formed on each side in symmetry and both slits have center portions thereof in the longitudinal direction thereof arranged close to each other and other portions thereof gradually parted away corresponding to the distance from the center portions.

5. An absorptive product according to any one of claims 1 to 4, wherein the resilient body is formed of a film-like resilient body having a given width which imparts a contracting force mainly in the longitudinal direction.

6. An absorptive product according to any one of claims 1 to 5, wherein the absorbent is formed by stacking a first absorbent layer having high liquid diffusivity of the absorbed liquid and a second absorbent layer having high liquid holding property of the absorbed liquid, the resilient body is fixed to the second absorbent layer side, and the above-mentioned slits are formed in the second absorbent layer.

7. An absorptive product according to claim 6, wherein a notched portion is formed in the first absorbent layer corresponding to a position where the resilient body is formed.
